# EUROPEAN PATENT SPECIFICATION

(11) **EP 0 818 462 B1**
(45) Date of publication and mention of the grant of the patent: **12.10.2005**
(21) Application number: 96906117.5
(22) Date of filing: 28.02.1996
(51) Int. Cl.: C07K 5/06, C07K 5/10, C07K 5/02

(54) **GLU-TRP COMPRISING TRIPEPTIDES**
GLU-TRP ENTHALTENDE TRIPEPTIDE
TRIPEPTIDES CONTENANT GLU-TRP

(30) Priority: 02.03.1995 RU 95102461
(43) Date of publication of application: 14.01.1998
(73) Proprietor: Immunotech Developments Inc., Toronto, Ontario M2N 4J2 (CA)
(72) Inventor: DEIGIN, Vladislav Isakovich, Toronto, Ontario Canada M2N 4J2 (CA); KOROTKOV, Andrei Marxovich, Moscow, 121609 (RU)
(74) Representative: Benedum, Ulrich Max
(86) International application number: PCT/RU1996/000046
(87) International publication number: WO 1996/026955

(56) References cited:
- EP-A- 0 148 133
- EP-A- 0 346 501
- EP-A- 0 832 900
- WO-A-94/24154
- WO-A-95/03067
- CH-A- 654 841
- DE-A- 4 014 230
- GB-A- 2 109 796
- SU-A- 1 277 903
- US-A- 4 619 916

## Description

The invention relates to peptides and methods for obtaining biologically active substances and compositions with immuno-regulatory properties which can be used in medicine, veterinary science and experimental biochemistry.

Thymus extracts are widely used in applied medicine as immuno-regulators, specifically the so-called thymosin fraction 5 (see CH-A-659586, "Thymolin", by Goldstein A.L., Guna A., Latz M.M., Hardy H.A., White A.). These extracts are obtained from a polypeptide digest. However, the yield from those natural sources is low due to the complexity of the manufacturing process. Furthermore, the yields are not only low in terms of quantity but also the extracts are not consistent with regard to their physiochemical and biological properties. Side effects were observed in patients whom polypeptides from natural sources were administered.

There are nowadays synthetic peptide chains available which have immuno-regulatory properties (PCT-WO/089/06134, EP-A-230052, US-A-5 008 246 and US-A-5 013 723). WO 95/03067 and EP-A-0 346 501 disclose pharmaceutical peptide preparations that contain L-Lys-L-Glu or L-Glu-L-Trp as active ingredients for inducing a heightened state of anti-microbial cellular or humoral activity and treatment of a immunodeficient status. These synthetic peptide compositions contain a limited number of essential ingredients, have high activity and low toxicity and are usually void of side effects which make them suitable for medical use.

It is therefore an object of the invention to provide synthetic peptides which have equally good or improved biological properties in comparison to the above-mentioned peptide extracts from natural sources.

This object is solved by providing peptides having the following general formula:

X-Glu-Trp-Y,

wherein X represents H, Gly, Ala, Leu, Ile, Val, NVal, Pro, Tyr, Phe, Trp, D-Ala, D-Leu, D-Ile, D-Val, DNVal, D-Pro, D-Tyr, D-Phe, D-Trp, Y-aminobuteric acid or ζ-aminocapronic acid; and Y represents Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid, ζ-aminocapronic acid, -OH or a (C₁-C₃) mono or disubstituted amide.

A further object of the invention is to provide a technological process for the synthesis of a peptide, which is easy to perform and provides the peptide in high yield and in good quality.

The essence of the new method is the synthesis of the new peptide which takes place in a solution by the successive growth of the chain from the C-terminal end of the molecule, using a strategy of maximally blocking the functional groups, starting from amino acid alkyl ether, using the method of activating the ethers and the method of mixing anhydrides using *tert*-butyloxycarbonyl amino acid.

The following table 1 shows the R_{f}-values of the peptide chains obtained by using: for R_{f1} (chloroform-methanol-32% acetic acid = 60:45:20) and for R_{f2} (butanol-pyridine-acetic acid = 5:5:4:1)

**TABLE 1**

| **PEPTIDE** | **R**_{**f1**} | **R**_{**f2**} |
|---|---|---|
| Abu-Glu-Trp-OH | 0.40 | 0.56 |
| Aca-Glu-Trp-OH | 0.41 | 0.57 |
| Ala-Glu-Trp-NH₂ | 0.40 | 0.51 |
| Arg-Glu-Trp-OH | 0.26 | 0.48 |
| D-Ala-Glu-Trp-OH | 0.37 | 0.55 |
| D-Ile-Glu-Trp-D-Phe | 0.71 | 0.77 |
| D-Ile-Glu-Trp-OH | 0.39 | 0.54 |
| D-Leu-Glu-Trp-NH₂ | 0.35 | 0.56 |
| D-Leu-Glu-Trp-OH | 0.37 | 0.57 |
| D-NVal-Glu-Trp-OH | 0.38 | 0.56 |
| D-Phe-Glu-Trp-Ala | 0.69 | 0.76 |
| D-Pro-Glu-Trp-OH | 0.58 | 0.72 |
| D-Trp-Glu-Trp-OH | 0.47 | 0.56 |
| D-Tyr-Glu-Trp-OH | 0.45 | 0.57 |
| D-Val-Glu-Trp-NH₂ | 0.43 | 0.53 |
| Gly-Glu-Trp-Gly | 0.44 | 0.49 |
| Gly-Glu-Trp-OH | 0.42 | 0.56 |
| H-Glu-Trp-Abu | 0.49 | 0.54 |
| H-Glu-Trp-Aca | 0.51 | 0.56 |
| H-Glu-Trp-Arg | 0.28 | 0.40 |
| H-Glu-Trp-D-Ala | 0.61 | 0.70 |
| H-Glu-Trp-D-Ile | 0.63 | 0.71 |
| H-Glu-Trp-D-Leu | 0.64 | 0.72 |
| H-Glu-Trp-D-NVal | 0.65 | 0.69 |
| H-Glu-Trp-D-Pro | 0.66 | 0.69 |
| H-Glu-Trp-D-Trp | 0.63 | 0.66 |
| H-Glu-Trp-D-Tyr | 0.61 | 0.66 |
| H-Glu-Trp-D-Val | 0.65 | 0.71 |
| H-Glu-Trp-Ile | 0.64 | 0.68 |
| H-Glu-Trp-Gly | 0.54 | 0.58 |
| H-Glu-Trp-NH₂ | 0.42 | 0.55 |
| H-Glu-Trp-N₂H₃ | 0.32 | 0.41 |
| H-Glu-Trp-NVal | 0.67 | 0.71 |
| H-Glu-Trp-Trp | 0.64 | 0.67 |
| H-Glu-Trp-Tyr | 0.62 | 0.66 |
| H-Glu-Trp-Val | 0.66 | 0.71 |
| His-Glu-Trp-OH | 0.31 | 0.58 |
| Ile-Glu-Trp-Phe | 0.71 | 0.78 |
| Ile-Glu-Trp-OH | 0.38 | 0.54 |
| Ile-Glu-Trp-Phe | 0.72 | 0.78 |
| Ile-Glu-Trp-Pro | 0.68 | 0.81 |
| Leu-Glu-Trp-OH | 0.39 | 0.56 |
| Lys-Glu-Trp-OH | 0.30 | 0.51 |
| Lys-Glu-Trp-Tyr | 0.32 | 0.50 |
| NVal-Glu-Trp-OH | 0.37 | 0.55 |
| Phe-Glu-Trp-NH₂ | 0.53 | 0.68 |
| Pro-Glu-Trp-Leu | 0.67 | 0.75 |
| Pro-Glu-Trp-OH | 0.59 | 0.72 |
| Trp-Glu-Trp-OH | 0.48 | 0.59 |
| Tyr-Glu-Trp-OH | 0.46 | 0.58 |
| Val-Glu-Trp-Ala | 0.61 | 0.71 |
| Val-Glu-Trp-NH₂ | 0.38 | 0.52 |
| Val-Glu-Trp-OH | 0.36 | 0.51 |
| Val-Glu-Trp-Tyr | 0.59 | 0.61 |

The peptides with the above formulas have the texture of a powder. They have a white colour and are soluble in water, partially soluble in alcohol and non-soluble in chloroform.

The invention now is illustrated by the following example.

The example refers to a method for synthesizing a peptide having the formula

H-Ile-Glu-Trp-OH,

which method comprises the steps of:

### 1. Obtaining Boc-Ile-OPFP:

a mixture was prepared comprising 46.0 g (0.2 mole) Boc-Ile-OH and 40.5 g (0.22 mole) pentafluorophenole in 100 ml ethylacetate, chilled to -5° C and 45.3 g (0.22 ml) N,N-dicyclohexylcarbodiimide was added. The mixture was then stirred at room temperature for three hours. The dicyclohexylcarbamide was filtered out, the solubles were evaporated under vacuum and the remains were crystallized in a mixture of ethyl acetate/hexane. The residue is then obtained by filtration. The yield was 71.3 g (90%).

### 2. Obtaining Boc-Ile-Glu-Trp-OH:

19.8 g (0.05 mole) Boc-Ile-OPFP was dissolved in 100 ml dimethylformamide and, while stirring, 20 g (0.06 mole) Glu-Trp and 5.0 g (0.06 mole) NaHCO₃ dissolved in water were added. The solution was stirred at room temperature for 20 hours. The solvents where evaporated in vacuum. To the remains there wre added 200 ml ethyl acetate and 200 ml 2% sulphuric acid (2 x 100 ml), enriched with NaCl solution to pH = 7. Then the remains were dried with dehydrated sodium sulphate. The solvents were then removed under vacuum. The remains where crystallized in ethyl acetate hexane, filtered and dried under vacuum. The yield was 20.5 g (75%).

### 3. Obtaining H-Ile-Glu-Trp-OH:

20.5 g Boc-Ile-Glu-Trp-OH was dissolved in 150 ml formic acid, stirred for 3.5 hours at 45° C and the solvent evaporated under vacuum. 200 ml water was added to the residue and the process of evaporation under vacuum was repeated. 300 ml isopropyl and 200 ml ether were added to the residue and left to cure for 10 hours. The residue was filtered and dried under vacuum to give a yield of 15.3 g (75%).

The peptide was purified by reversed-phase chromatography in acetonitrile 0.1% in a solution of trifluoroacetic acid to give a yield of 13 g (85%).

The peptide showed the following characteristic physiochemical properties:

| | |
|---|---|
| Sequence | H-Ile-Glu-Trp-OH |
| Total formula | C₂₄-H₃₀-N₄-O₄ |
| Molecular weight | 446.5 Da |
| Visual texture | white powder with a shade of |
| | yellow or grey powder. |
| Solubility | soluble in water, non-soluble in |
| | chloroform. |
| UV - spectrum in the range of 250 - 300 nm | peaks at 280±2 nm 287±2 nm. |

The biological activity of the new peptide was studied on guinea pigs using recognised testing methods of E-yield of lymphocytes of the guinea pigs subsequent to processing with trypsin.

| QUANTITY E-POK,(%) | | | | | | | | | |
|---|---|---|---|---|---|---|---|---|---|
| Compound | Untreated animals | Treated with trypsin | After treatment with trypsin and compound with concentration mg/ml* | | | | | | |
| | | | 10⁻⁶ | 10⁻⁷ | 10⁻⁸ | 10⁻⁹ | 10⁻¹⁰ | 10⁻¹¹ | 10⁻¹² |
| Tymolin | 66.5 | 36.1 | 57.0 | 40.1 | 37.0 | 35.3 | 37.4 | 36.5 | 34.7 |
| Tymozine Fraction 5 | 66.5 | 36.1 | 60.3 | 35.4 | 33.4 | 39.5 | 39.1 | 33.7 | 35.8 |
| Ile-Glu-Trp | 66.5 | 36.1 | 61.4 | 63.9 | 64.8 | 60.2 | 37.5 | 40.0 | 34.3 |

| | | | | | | | | | |
|---|---|---|---|---|---|---|---|---|---|
| * Each concentration was tested on 5 animals. The positive growth differs in relation to the control group E-POK is 50% or higher. | | | | | | | | | |

This shows that *in vitro* the activity of peptide of the invention is 10³ times higher than the activity of other known compounds.

To establish the safety of use of the peptide, toxicity tests where performed. Toxicity tests where performed according to guidelines set out by the Pharmacological committee PF "Guidelines for the pre clinical study general toxic activity of new pharmalogical compounds" M., 1985.

The results of the experiments show that intravenous injections of a 1/1000 dose of the peptide did not exhibit any toxic activity and LD₅₀ was not reached.

The peptide which possesses biologically active properties can be used in medicine and veterinary science.

## Claims

1. A peptide having the formula
X-Glu-Trp-Y,
wherein X represents H, Gly, Ala, Leu, Ile, Val, NVal, Pro, Tyr, Phe, Trp, D-Ala, D-Leu. D-Ile, D-Val, D-NVal, D-Pro, D-Tyr, D-Phe, D-Trp, γ-aminobuteric acid or ζ-aminocapronic acid; and
Y represents Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid, ζ-aminocapronic acid, -OH or a mono- or di-(C₁-C₃)-substituted amide.

2. A method for preparing a peptide having the formula:
X-Glu-Trp-Y,
wherein X represents H, Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid or ζ-aminocapronic acid, and
Y represents Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid, ζ-aminocapronic acid, -OH or a mono- or di-(C₁-C₃)-substituted amide,
wherein the peptide is synthesized in solution by the successive growth of a chain from the C-terminus of the molecule, **characterized by** blocking the functional groups, starting from amino acid alkyl ethers, the method of activating the ethers, the method of mixed anhydrides using *tert*-butyloxycarbonyl amino acids, processing with formic acid and further purification by crystallization, to obtain a product having the formula X-Glu-Trp-Y, which is then further purified by reverse-phase chromatography.

3. Use of a compound according to claim 1 as an active substance for preparing a drug for use in medicine.

4. Use of a compound according to claim 1 for preparing a pharmaceutical composition having immunoregulating properties for treating immunological disorders.

5. A method of preparing a pharmaceutical composition with immunoregulating properties, **characterized in that** a peptide having the formula
X-Glu-Trp-Y,
wherein X represents H, Gly, Ala, Leu, Ile, Val, NVal, Pro, Tyr, Phe, Trp, D-Ala, D-Leu. D-Ile, D-Val, D-NVal, D-Pro, D-Tyr, D-Phe, D-Trp, γ-aminobuteric acid or ζ-aminocapronic acid, and
Y represents Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid, ζ-aminocapronic acid, -OH or a mono- or di-(C₁-C₃)-substituted amide,
is synthesized in solution by a successive growth of a chain from the C-terminus of the molecule and further purified by crystallization and reverse-phase chromatography.

6. Use of a peptide having the formula
X-Glu-Trp-Y,
wherein X represents H, Gly, Ala, Leu, Ile, Val, NVal, Pro, Tyr, Phe, Trp, D-Ala, D-Leu. D-Ile, D-Val, D-NVal, D-Pro, D-Tyr, D-Phe, D-Trp, γ-aminobuteric acid or ζ-aminocapronic acid; and
Y represents Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-aminobuteric acid, ζ-aminocapronic acid, -OH or a (C₁ - C₃) mono- or disubstituted amide,
for preparing a drug for use in medicine.

7. Use of a peptide according to claim 6, wherein the peptide has the formula
X-Ile-Glu-Trp-OH.

## Patentansprüche

1. Peptid der Formel
X-Glu-Trp-Y,
worin ist:
X gleich H, Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure, ξ-Aminocapronsäure; und
Y gleich Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure, ξ-Aminocapronsäure, -OH oder ein Mono- oder Di-(C₁-C₃)-substituiertes Amid.

2. Verfahren zur Herstellung eines Peptids der Formel
X-Glu-Trp-Y,
worin ist:
X gleich H, Gly, Ala, Leu, lle, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure oder ξ-Aminocapronsäure, und
Y gleich Gly, Ala, Leu, Ile, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure, ξ-Aminocapronsäure, -OH oder ein Mono- oder Di-(C₁-C₃)-substituiertes Amid,
wobei das Peptid in Lösung durch sukzessive Kettenverlängerung am C-Terminus des Moleküls synthetisiert wird, **gekennzeichnet durch** Schützen funktioneller Gruppen; das Verfahren aktivierter Ether, ausgehend von Aminosäurealkylethern; das Verfahren gemischter Anhydride unter Verwendung von tert-Butyloxycarbonylaminosäuren; die Behandlung mit Ameisensäure und die weitere Aufreinigung **durch** Kristallisation, so dass man ein Produkt erhält der Formel X-Glu-Trp-Y, das dann **durch** Reverse-Phase-Chromatographie weiter aufgereinigt wird.

3. Verwendung einer Verbindung nach Anspruch 1 als Wirksubstanz zur Herstellung eines Arzneimittels zur Verwendung in der Medizin.

4. Verwendung einer Verbindung nach Anspruch 1, zur Herstellung einer pharmazeutischen Zusammensetzung mit immunregulierenden Eigenschaften zur Behandlung von Immunkrankheiten.

5. Verfahren zur Herstellung einer pharmazeutischen Zusammensetzung mit immunregulierenden Eigenschaften, **gekennzeichnet durch** ein Peptid der Formel
X-Glu-Trp-Y,
worin ist:
X gleich H, Gly, Ala, Leu, Ile, Val, NVal, Pro, Tyr, Phe, Trp, D-Ala, D-Leu, D-Iie, D-Val, D-NVal, D-Pro, D-Tyr, D-Phe, D-Trp, γ-Aminobuttersäure oder ξ-Aminocapronsäure, und
Y gleich Gly, Ala, Leu, lle, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure, ξ-Aminocapronsäure, -OH oder ein Mono- oder Di-(C₁-C₃)-substituiertes Amid,
das in Lösung **durch** sukzessive Kettenverlängerung am C-Terminus des Moleküls synthetisiert wird und weiteres Aufreinigen **durch** Kristallisation und Reverse-Phase-Chromatographie.

6. Verwendung eines Peptides der Formel
X-Glu-Trp-Y,
worin ist:
X gleich H, Gly, Ala, Leu, lle, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure oder ξ-Aminocapronsäure, und
Y gleich Gly, Ala, Leu, lle, Val, NVal, Pro, Tyr, Phe, Trp, D-Ala, D-Leu, D-Ile, D-Val, D-NVal, D-Pro, D-Tyr, D-Phe, D-Trp, γ-Aminobuttersäure, ξ-Aminocapronsäure, -OH oder ein Mono- oder Di-(C₁-C₃)-substituiertes Amid, zur Herstellung eines Arzneimittels für die Medizin.

7. Verwendung eines Peptids nach Anspruch 6, wobei das Peptid die Formel
X-Ile-Glu-Trp-OH
besitzt.

## Revendications

1. Un peptide ayant la formule suivante
X- Glu - Trp-Y,
dans laquelle formule X represente H, Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique ou acide ζ-aminocapronique, et
Y represente Gly, Ala, Leu, Ile, Val, Nval, Pro, Tyr, Phe, Trp, D-Ala, D-Leu, D-Ilem D-Val, D-Nval, D-Pro, D-Tyr, D-Phe, D-Trp, acide γ-aminobuterique ou acide ζ-aminocapronique, -OH ou un mono- ou di- amide- (C1-C3) substitue.

2. Un procede pour preparer un peptide ayant la formule suivante :
X-Glu-Trp-Y,
dans laquelle X représente H, Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique ou acide ζ-aminocapronique, et
Y represente Gly, Ala, Leu, Ile, Val, Nval, Pro, Tyr, Phe, Trp, D-Ala, D-Leu, D-Ilem D-Val, D-Nval, D-Pro, D-Tyr, D-Phe, D-Trp, acide γ-aminobuterique ou acide ζ-aminocapronique, -OH ou un mono- ou di- amide- (C1-C3) substitue,
ou le peptide est synthetise en solution grace a la croissance successive d'une chaine a partir de l'extremite C de la molecule, **caracterise par** le blocage des groupes fonctionnels, par le procede d'activation des ethers commencant avec des ethers alkyliques de l'acide amine, par le procede des anhydrides melanges en utilisant les acides amines de tert-butyloxycarbonyl, par le traitement avec l'acide formique et une purification supplementaire par cristallisation pour obtenir un produit ayant la formule X-Glu-Trp-Y, lequel produit est ensuite purifie davantage par la chromatographie a phase inversee.

3. Utilisation d'un compose selon la revendication 1 en tant que substance active pour preparer un medicament destine a etre utilise en medecine.

4. Utilisation d'un compose selon la revendication 1 pour préparer une compositiion pharmaceutique ayant des proprietes immunoregulatrices destinee au traitement des maladies immunologiques.

5. Un procede pour preparer une composition pharmaceutique avec des proprietes immunoregulatrices, **caracterise en ce qu'**un peptide ayant la formule :
X-Glu-Trp-Y,
dans laquelle X représente represente H, Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique
ou acide ζ-aminocapronique, et
Y représente Gly, Ala, Leu, Ile, Val, Nval, Pro, Tyr, Phe, Trp, D-Ala, D-Leu, D-Ilem D-Val, D-Nval, D-Pro, D-Tyr, D-Phe, D-Trp, acide γ-aminobuterique ou acide ζ-aminocapronique, et
Y représente Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique ou acide ζ-aminocapronique, -OH ou un un mono- ou di- amide- (C1-C3) substitue
est synthetise dans une solution grace a la croissance successive d'une chaine a partir d'une extremite C de la molecule et purifie davantage par cristallisation et par la chromatographie a phase inversee,

6. Utilisation d'un peptide ayant la formule :
X-Glu-Trp-Y,
dans laquelle X represente represente H, Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique
ou acide ζ-aminocapronique, et
Y represente Gly, Ala, Leu, Ile, Val, Nval, Pro, Tyr, Phe, D-Ala, D-Leu, D-Ile, D-Val, D-Nval, D-Pro, D-Tyr, D-Phe, D-Trp, acide γ-aminobuterique ou acide ζ-aminocapronique, -OH ou un un mono- ou di- amide- (C1-C3) substitue
pour la préparation d'un médicament destine a etre utilise en medecine.

7. Utilisation d'un peptide selon la revendication 6, ou le peptide a la formule :
X-Ile-Glu-Trp-OH.
